# EUROPEAN PATENT APPLICATION

(11) **EP 4 790 977 A1**
(43) Date of publication of application: **12.08.2026**
(21) Application number: 26156826.5
(22) Date of filing: 06.02.2026
(51) Int. Cl.: H04N 7/14

(54) **ADAPTIVE COMMUNICATION SUPPORT SYSTEM FOR COGNITIVE IMPAIRMENTS**

(30) Priority: 06.02.2025 US 202519047595
(71) Applicant: Mitel Networks Corporation, Ottawa, Ontario K2K 3K1 (CA)
(72) Inventor: Murthy, Tejas, 56008 Bangalore (IN); Radhakrishnan, Jayachandran, 560035 Bengaluru (IN); Naidoo, Logendra, K1S 0W8 Ottawa (IN)
(74) Representative: McDougall, James

(57) **Abstract**

Systems and methods including one or more processors and one or more non-transitory storage devices storing computing instructions configured to run on the one or more processors and perform acts of receiving, via the one or more sensors, real-time physiological and behavioral data of the user; analyzing, using a machine learning model, the physiological and behavioral data to determine a cognitive or sensory state of the user; identifying, based on the determined cognitive or sensory state, one or more specific needs of the user; and modifying, based on the one or more identified specific needs, one or more digital environment elements of the communication apparatus to address the cognitive or sensory state of the user. Other embodiments are disclosed herein.

## Description

### TECHNICAL FIELD

This disclosure relates generally to videoconferencing methods and systems and more particularly to methods and systems for altering videoconferencing feeds in view of detected historical and in-session health data of participants in the videoconference.

### BACKGROUND

In modern videoconferencing systems, such as those used in virtual meetings, online events, and remote collaboration, participant engagement for individuals with cognitive impairments often poses a significant challenge. Previous approaches to addressing cognitive aging and communication support have often relied on separate tools rather than a cohesive system. For example, memory aids have typically been delivered through reminder apps, wearable devices, or cognitive training platforms. Some platforms may offer cognitive exercises designed to enhance memory and attention. Assistive technologies, such as voice assistants, can set reminders or answer basic questions. Emotion detection and stress management have usually been managed by standalone emotion recognition apps or wearable devices, which monitor stress levels or offer relaxation prompts. These previous solutions are generally not personalized, lack real-time adaptability, and do not comprehensively address the combined cognitive and emotional challenges that arise during communication. As a result, these approaches often fall short in providing the necessary personalized support, thus hampering effective communication and collaboration.

Efforts to address these issues have included static accessibility settings and basic software for people with cognitive impairments, such as screen readers, text-to-speech options, and color contrast adjustments. While these tools can address some of the needs of individuals with cognitive impairments, they often are largely static and do not adapt in real-time to the changing conditions of users with cognitive impairments. Further, these platforms lack integration with tools that can dynamically adjust to fluctuations in cognitive abilities or provide real-time customization based on user feedback, making them insufficient for users needing more specialized support.

Therefore, there is a need for improved methods and systems to provide tailored assistance and enhance the digital experience for users with cognitive impairments.

### BRIEF DESCRIPTION OF THE DRAWINGS

The subject matter of the present disclosure is particularly pointed out and distinctly claimed in the concluding portion of the specification. A more complete understanding of the present disclosure, however, may best be obtained by referring to the detailed description and claims when considered in connection with the drawing figures, wherein like numerals denote like elements and wherein:
FIG. 1 is a block diagram of a system according to aspects of this disclosure;
FIG. 2 is a block diagram of a method for dynamically adapting a communication apparatus based on detected health data according to aspects of this disclosure;
FIG. 3 is a block diagram depicting various methods for providing assistance to a user experiencing cognitive strain according to aspects of this disclosure;
FIG. 4 is a flowchart for dynamically adapting a communication apparatus based on detected health data according to aspects of this disclosure;
FIG. 5 is a flowchart for generating prompts for a user based on the user's mental state according to aspects of this disclosure; and
FIG. 6 illustrates a representative block diagram of a computer system, according to an embodiment.

It will be appreciated that elements in the figures are illustrated for simplicity and clarity and have not necessarily been drawn to scale. For example, the dimensions of some of the elements in the figures may be exaggerated relative to other elements to help to improve understanding of illustrated embodiments of the present invention.

### DETAILED DESCRIPTION

The description of exemplary embodiments of the present invention provided herein is merely exemplary and is intended for purposes of illustration only; the following description is not intended to limit the scope of the invention as claimed. Moreover, recitation of multiple embodiments having stated features is not intended to exclude other embodiments having additional features or other embodiments incorporating different combinations of the stated features.

It must also be noted that, the term "exemplary" is used in the sense of "example," rather than "ideal."

It must also be noted that, as used in the specification and the appended claims, the singular forms "a," "an" and "the" include plural referents unless the context clearly dictates otherwise.

By "comprising" or "containing" or "including" it is meant that at least the named compound, element, particle, or method step is present in the composition or article or method, but does not exclude the presence of other compounds, materials, particles, method steps, even if the other such compounds, material, particles, method steps have the same function as what is named.

Relative terms, such as "about," "substantially," or "approximately" are used to include small variations with specific numerical values (e.g., +/- x%,), as well as including the situation of no variation (+/-0%). In various embodiments, the numerical value x is less than or equal to 10 - e.g., less than or equal to 5, to 2, to 1, or smaller.

As used herein, "database" refers to any suitable database for storing information, electronic files or code to be utilized to practice embodiments of this disclosure. As used herein, "server" refers to any suitable server, computer or computing device for performing functions utilized to practice embodiments of this disclosure.

As used herein, "software" refers to programs or other operating information utilized by a processor or other computing hardware.

As used herein, "meeting" means a meeting or conference such as telephonic, video, audio/video, in-person, a hybrid of any of the preceding, and any type of meeting involving multiple participants.

This disclosure provides a system for dynamically adapting digital communication and learning environments to cater to a wide spectrum of cognitive and sensory disabilities. Also, this disclosure provides a method for dynamically adapting digital communication and learning environments to support a wide range of cognitive and sensory disabilities. The digital communication and learning environments may be a communication apparatus, such as platforms like Zoom^{™}, Microsoft Teams^{™}, WebEx^{™}, and other similar applications. Leveraging artificial intelligence (AI) driven features, the system can continuously monitor a user's cognitive and sensory states while using the communication apparatus, allowing the system to make real-time adjustments to visual, auditory, and interactive elements of the communication apparatus. For example, the system can alter screen colors and contrast, highlight key information, use simple animations as applicable to the context, modulate audio pitches, and provide tailored prompts to maintain user engagement and reduce sensory discomfort. This method may be designed to support a broad range of disabilities, including but not limited to Attention-Deficit/Hyperactivity Disorder (ADHD), autism, visual and hearing impairments, dyslexia, and schizophrenia. The system described herein can also offer personalized memory aids, emotion-sensitive feedback, and predictive cognitive load management to help individuals with cognitive challenges like memory lapses, cognitive fatigue, and attention deficits.

The system may include a customizable cognitive companion that can adapt in real time to the user's communication environment. This companion can provide context-specific memory aids and integrate with adaptive memory exercises, such as personalized recall prompts and reminders, to help users navigate conversations more effectively. Additionally, the companion can offer emotion-sensitive interventions, adjusting its responses based on emotional cues to reduce cognitive overload and improve emotional well-being during interactions within the UC tool.

In some embodiments, the system may use Explainable Artificial Intelligence (XAI) to enhance transparency, which can provide clear explanations for artificial intelligence (AI)-driven actions like memory prompts and emotional interventions. This may enable users to understand the system's decisions, improving both engagement and satisfaction. The predictive cognitive load management feature may use real-time data and behavioral patterns to forecast cognitive strain, proactively intervening to minimize distractions and reduce cognitive fatigue. Overall, the system can dynamically adapt to the user's cognitive and emotional states while using a UC tool, offering personalized, real-time support for improved communication and cognitive health.

For users with ADHD, the system can identify moments of distraction or disengagement during virtual meetings and dynamically modify visual and auditory elements to recapture and maintain focus. For example, it can change the color scheme, highlight important information, use simple animations as applicable to the context, and provide subtle auditory cues to draw attention back to the task at hand. For individuals with autism, the system can adjust the pitch and tone of voices in communication sessions to avoid triggering discomfort, and it can use simplified visuals or emojis instead of text to enhance understanding. Additionally, the system may include customizable social stories and visual schedules that adjust in real-time based on user interactions and routines and can gather feedback on the different visual or auditory cues of the user, learn the cues, and dynamically personalize the individual's specific preference.

In educational settings, the system can offer personalized study prompts and interactive AR lessons within the communication apparatus, dynamically adapting the content's difficulty and format based on the student's progress and cognitive load. The system can predict when a student is becoming overwhelmed and suggest breaks or alternative learning strategies. This method can provide a consistent and predictive approach to accommodating diverse cognitive needs, making digital communication, and learning environments more inclusive and effective for individuals with ADHD, autism, and other cognitive impairments.

Previous solutions for aiding individuals with cognitive impairments in digital environments have primarily relied on static accessibility settings and second-rate software for people with cognitive impairments. Existing popular collaboration platforms offer basic features like screen readers, text-to-speech options, and color contrast adjustments. However, these features are largely static and do not adapt in real-time to the changing conditions of users with cognitive impairments. For instance, these platforms lack integration with tools that can dynamically adjust to fluctuations in cognitive abilities or provide real-time customization based on user feedback, making them insufficient for users needing more specialized support. Also, many existing tools fail to meet the guidelines set by various standardization organizations, such as European Telecommunications Standards Institute (ETSI) EN 601 549, which ensures that Information and Communication Technology (ICT) products are adaptable and accessible in real-time, thus limiting their effectiveness for users with cognitive disabilities.

In addition, some third-party applications and tools are designed to assist users with specific conditions such as ADHD or autism, yet they often do not seamlessly integrate with mainstream communication and learning platforms. This disconnect limits their practical utility, as users must switch between different applications to access necessary support, disrupting workflow and reducing overall efficiency. The customization options offered by existing tools are typically limited to pre-set configurations and do not accommodate the nuanced needs of special-needs users dynamically throughout a collaboration session.

According to the World Health Organization (WHO), more than one-billion people worldwide live with some form of disability, representing about 15% of the global population, with a substantial portion experiencing cognitive impairments that affect their ability to use digital technologies effectively. Therefore, there is a need to address the needs of individuals with cognitive impairments using communication tools.

There are various standards like ETSI EG 202 116, ETSI EN 301 549, International Organization for Standardization and International Electrotechnical Commission (ISO/IEC) 29138-1:2009, International Organization for Standardization (ISO) 21801-1:2020, which focuses on ensuring that products and services are cognitively accessible, so that they can be easily used and understood by individuals with cognitive disabilities. These standards make it complex and critical for communication apparatus providers to adhere and add value to these standards so that all users can use the system effectively.

ETSI EG 202 116 focuses on identifying the specific needs of users with disabilities and provides recommendations for creating telecommunications products and services that can be used by everyone, regardless of their abilities. It covers various aspects such as physical accessibility, cognitive accessibility, and usability.

ETSI EN 301 549 specifies the accessibility requirements for Information and Communication Technology (ICT) products and services to ensure they are usable by people with disabilities.

ISO/IEC 29138-1:2009 categorizes various accessibility needs into different types of disabilities, including physical, sensory, and cognitive disabilities. It serves as a comprehensive resource for developers and designers to understand the specific needs of these user groups and to incorporate these considerations into the design and development of ICT products and services.

ISO 21801-1:2020 focuses on cognitive accessibility, providing guidelines for making products and services usable by people with cognitive impairments.

The systems and methods described herein may be in accordance with ETSI EG 202 116, ETSI EN 301 549 and related accessibility standards to improve inclusivity and adaptability in the communication system.

As described above, traditional "Accessibility" settings are typically static, configured once by users to address consistent environmental challenges. They generally do not adapt to changes in user conditions or content dynamics. In contrast, the systems and methods described herein can dynamically customize the interface, media stream, and content of a communication apparatus based on real-time analysis of the user's cognitive and sensory states. The system's architecture may adhere to ETSI EN 301 549, so that adjustments and customizations meet the prescribed accessibility requirements. This compliance allows the system to be both adaptable and accessible to users with diverse needs, including those with cognitive and sensory disabilities and can also be enhanced to be made adaptable to other standards like Institute of Electrical and Electronics Engineers (IEEE) P2794, ISO/IEC 29138-1.

The system described herein further provides a dynamic, real-time adaptive system that personalizes digital communication and learning environments based on the user's cognitive condition and engagement levels. Unlike static accessibility settings and specialized apps of existing solutions, the systems and methods described herein can continuously monitor and adjust visual, auditory, and interactive elements to meet the individual needs of users with ADHD, autism, or other cognitive impairments. This proactive approach can allow users to receive timely and context-aware support, such as focus prompts, customized visuals, and adjusted audio cues, enhancing their productivity and comfort. Existing solutions fail to offer this level of integration and adaptability, often providing only generic or one-time adjustments that do not effectively address the fluctuating cognitive states of these users. The systems and methods described herein can bridge this gap by offering a comprehensive and responsive solution that can transform digital experiences into more inclusive and effective environments for individuals with cognitive impairments.

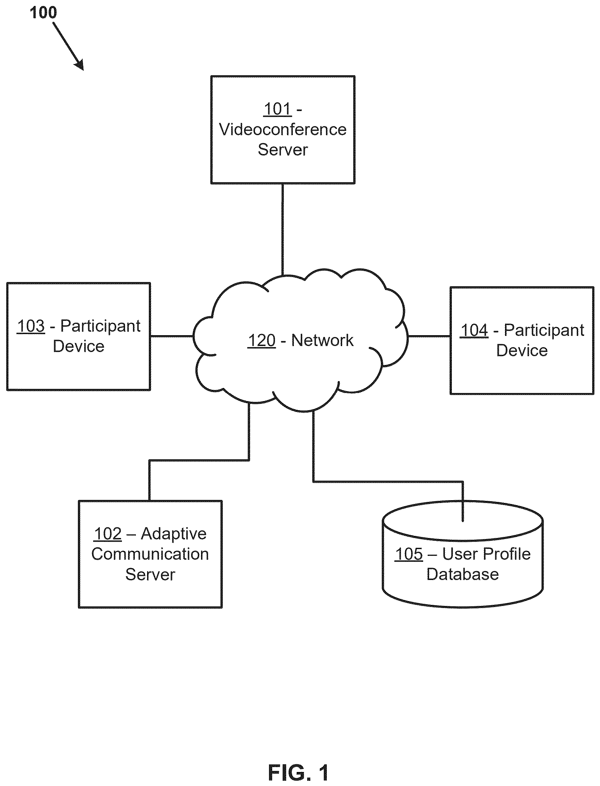

Turning to the figures, FIG. 1 illustrates a block diagram of a system 100 that can be employed for altering videoconference feeds, as described in greater detail below. System 100 is merely exemplary and embodiments of the system are not limited to the embodiments presented herein. System 100 can be employed in many different embodiments or examples not specifically depicted or described herein. In some embodiments, certain elements or modules of system 100 can perform various procedures, processes, and/or activities. In these or other embodiments, the procedures, processes, and/or activities can be performed by other suitable elements or modules of system 100.

Generally speaking, system 100 can be implemented with hardware and/or software. Part or all of the hardware and/or software implemented in system 100 can be conventional or part or all of the hardware and/or software can be customized (e.g., optimized) for implementing part or all of the functionality of system 100 described herein.

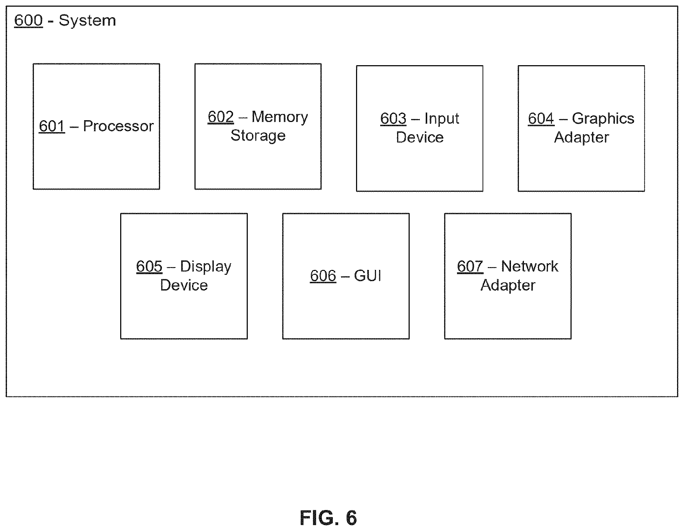

System 100 can include videoconference server 101, adaptive communication server 102, participant devices 103, 104, and/or user profile database 105. Videoconference server 101, adaptive communication server 102, and/or participant devices 103, 104 can each be a computer system, such as computer system 600 (FIG. 6), as described above, and can each be a single computer, a single server, a cluster or collection of computers or servers, or a cloud of computers or servers.

Participant devices 103, 104 can comprise any of the elements described in relation to computer system 600 (FIG. 6). For example, participant devices 103, 104 can be mobile devices. A mobile device can refer to a portable electronic device (e.g., an electronic device easily conveyable by hand by a person of average size) with the capability to present audio and/or visual data (e.g., text, images, videos, music, etc.). For example, a mobile electronic device can comprise at least one of a digital media player, a cellular telephone (e.g., a smartphone), a personal digital assistant, a handheld digital computer device (e.g., a tablet personal computer device), a laptop computer device (e.g., a notebook computer device, a netbook computer device), a wearable user computer device, or another portable computer device with the capability to present audio and/or visual data (e.g., images, videos, music, etc.). Thus, in many examples, a mobile electronic device can comprise a volume and/or weight sufficiently small as to permit the mobile electronic device to be easily conveyable by hand.

Exemplary mobile electronic devices can comprise (i) an iPod^{®}, iPhone^{®}, iTouch^{®}, iPad^{®}, MacBook^{®} or similar product by Apple Inc. of Cupertino, California, United States of America, (ii) a Pixel^{™} product or a similar product by Google Inc. of Menlo Park, California, United States of America, (iii) a Lumia^{®} or similar product by the Nokia Corporation of Keilaniemi, Espoo, Finland, and/or (iv) a Galaxy^{™} or similar product by the Samsung Group of Samsung Town, Seoul, South Korea. Further, in the same or different embodiments, a mobile electronic device can comprise an electronic device configured to implement one or more of (i) the iPhone^{®} operating system by Apple Inc. of Cupertino, California, United States of America, (ii) the Android^{™} operating system Google Inc. of Menlo Park, California, United States of America, (iii) the Palm^{®} operating system by Palm, Inc. of Sunnyvale, California, United States, (iv) the Android^{™} operating system developed by the Open Handset Alliance, (v) the Windows Mobile^{™} operating system by Microsoft Corp. of Redmond, Washington, United States of America, or (vi) the Symbian^{™} operating system by Nokia Corp. of Keilaniemi, Espoo, Finland.

Further still, the term "wearable device" as used herein can refer to an electronic device with the capability to present audio and/or visual data (e.g., text, images, videos, music, etc.) that is configured to be worn by a user and/or mountable (e.g., fixed) on the user of the wearable user computer device (e.g., sometimes under or over clothing; and/or sometimes integrated with and/or as clothing and/or another accessory, such as, for example, a hat, eyeglasses, a wrist watch, shoes, etc.). A wearable user computer device can comprise a mobile electronic device, and vice versa. However, a wearable user computer device does not necessarily comprise a mobile electronic device, and vice versa.

In specific examples, a wearable user computer device can comprise a head mountable wearable user computer device (e.g., one or more head mountable displays, one or more eyeglasses, one or more contact lenses, one or more retinal displays, etc.) or a limb mountable wearable user computer device (e.g., a smart watch, smart ring, etc.). In these examples, a head mountable wearable user computer device can be mountable in close proximity to one or both eyes of a user of the head mountable wearable user computer device and/or vectored in alignment with a field of view of the user.

In more specific examples, a head mountable wearable user computer device can comprise (i) Google Glass^{™} product or a similar product by Google Inc. of Menlo Park, California, United States of America; (ii) the Eye Tap^{™} product, the Laser Eye Tap^{™} product, or a similar product by ePI Lab of Toronto, Ontario, Canada, and/or (iii) the Raptyr^{™} product, the STAR 1200^{™} product, the Vuzix Smart Glasses M100^{™} product, or a similar product by Vuzix Corporation of Rochester, New York, United States of America. In other specific examples, a head mountable wearable user computer device can comprise the Virtual Retinal Display^{™} product, or similar product by the University of Washington of Seattle, Washington, United States of America. Meanwhile, in further specific examples, a limb mountable wearable user computer device can comprise the iWatch^{™} product, or similar product by Apple Inc. of Cupertino, California, United States of America, the Galaxy Gear or similar product of Samsung Group of Samsung Town, Seoul, South Korea, the Moto 360 product or similar product of Motorola of Schaumburg, Illinois, United States of America, and/or the Zip^{™} product, One^{™} product, Flex^{™} product, Charge^{™} product, Surge^{™} product, or similar product by Fitbit Inc. of San Francisco, California, United States of America.

Videoconference server 101, adaptive communication server 102, and/or one or more of participant devices 103, 104 can each comprise one or more input devices (e.g., one or more keyboards, one or more keypads, one or more pointing devices such as a computer mouse or computer mice, one or more touchscreen displays, a microphone, etc.), and/or can each comprise one or more display devices (e.g., one or more monitors, one or more touch screen displays, projectors, etc.). In these or other embodiments, one or more of the input device(s) can be similar or identical to input device 603 (FIG. 6). Further, one or more of the display device(s) can be similar or identical to display device 605 (FIG. 6). The input device(s) and the display device(s) can be coupled to the processing module(s) and/or the memory storage module(s) of videoconference server 101, adaptive communication server 102, and/or one or more of participant devices 103, 104 in a wired manner and/or a wireless manner, and the coupling can be direct and/or indirect, as well as locally and/or remotely. As an example of an indirect manner (which may or may not also be a remote manner), a keyboard-video-mouse (KVM) switch can be used to couple the input device(s) and the display device(s) to the processing module(s) and/or the memory storage module(s). In some embodiments, the KVM switch also can be part of videoconference server 101, adaptive communication server 102, and/or one or more of participant devices 103, 104. In a similar manner, the processing module(s) and the memory storage module(s) can be local and/or remote to each other.

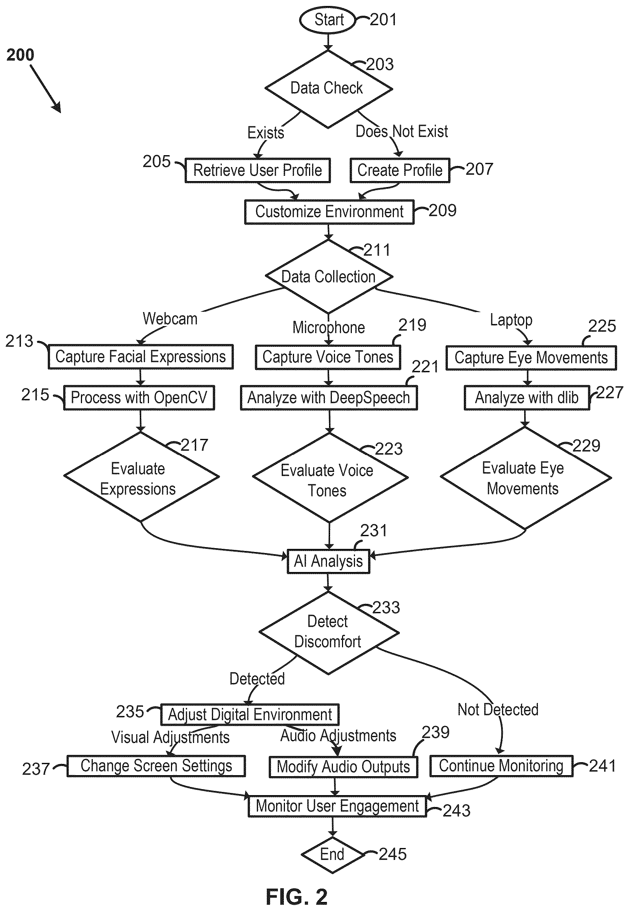

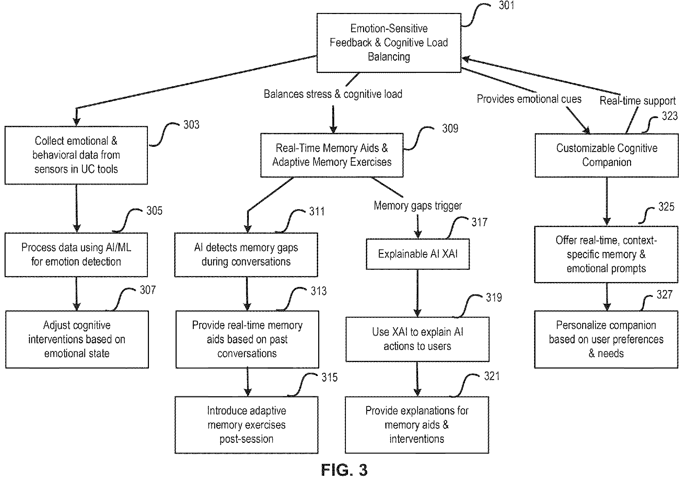

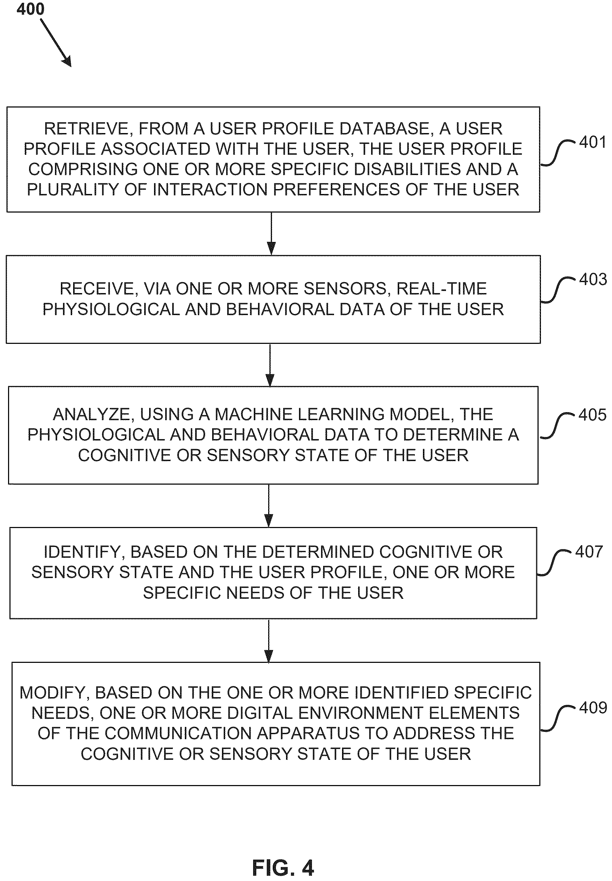

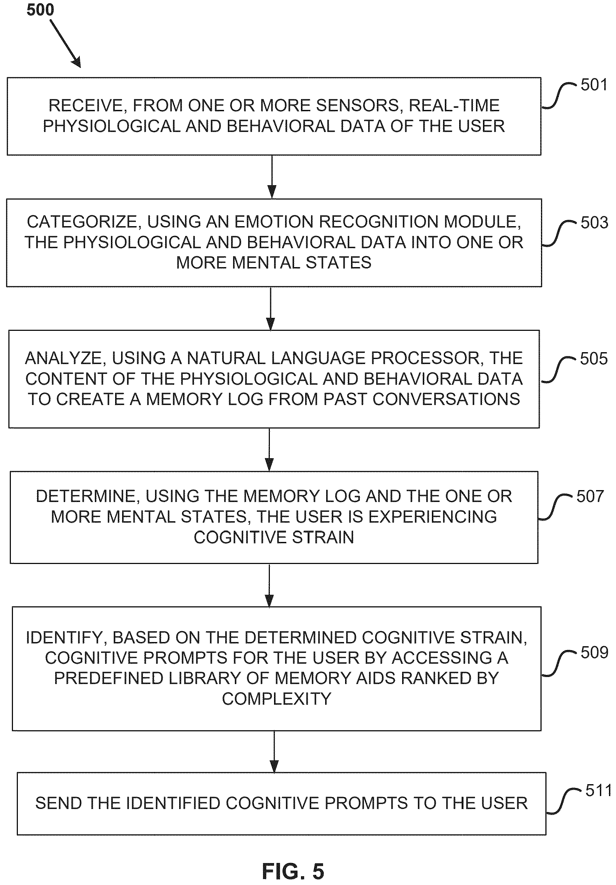

Videoconference server 101 can host and/or run one or more videoconference software platforms. Adaptive communication server 102 can host a system for altering videoconferences as described herein. For example, adaptive communication server 102 can perform one or more steps of method 200 (FIG. 2), FIG. 3, method 400 (FIG. 4), and/or method 500 (FIG. 5). In some embodiments, adaptive communication server 102 can be embodied in and/or distribute a software application capable of performing one or more steps of method 200 (FIG. 2), FIG. 3, method 400 (FIG. 4), and/or method 500 (FIG. 5). The software application can be installed/installable on one or more of participant devices 103, 104.

Videoconference server 101, adaptive communication server 102, and/or participant devices 103, 104 can communicate or interface (e.g., interact) with one another through network 120. Network 120 can be an intranet that is not open to the public, a mesh network of individual systems, and/or a distributed system. Accordingly, in many embodiments, videoconference server 101 and/or adaptive communication server 102 (and/or the software used by such systems) can refer to a back end of system 100 operated by an operator and/or administrator of system 100, and participant devices 103, 104 (and/or the software used by such systems) can refer to a front end of system 100 used by one or more participants, respectively. An operator and/or administrator of system 100 can manage system 100, the processing module(s) of system 100, and/or the memory storage module(s) of system 100 using the input device(s) and/or display device(s) of system 100.

Videoconference server 101, adaptive communication server 102, and/or participant devices 103, 104 also can be configured to communicate with one or more databases. The one or more databases may include the user profile database 105. The user profile database 105 may include one or more user profiles for each user of the communication apparatus, where each profile in the user profile database can contain specific disabilities and/or interaction preferences for each user of the communication apparatus. The one or more databases can also comprise a historical videoconference database that stores records about past videoconferences. A historical videoconference database can also comprise an interaction database containing information about interactions of participant devices with a videoconference. These interactions can be tied to a unique identifier (e.g., an IP address, an advertising ID, device ID, etc.) and/or a user account. In embodiments where a participant interacts with a videoconference before logging into a user account, data stored in the one or more database that is associated with a unique identifier can be merged with and/or associated with data associated with the user account. Data can be deleted from a database when it becomes older than a maximum age, which can be set by an administrator of system 100. Data collected in real-time can be streamed to a database for storage, thereby increasing a storage speed of a database.

The one or more databases can be stored on one or more memory storage modules (e.g., non-transitory memory storage module(s)), which can be similar or identical to the one or more memory storage module(s) (e.g., non-transitory memory storage module(s)) described above with respect to computer system 600 (FIG. 6). Further, the one or more databases can each be stored on a single memory storage module of the memory storage module(s), and/or the non-transitory memory storage module(s) storing the one or more databases or the contents of that particular database can be spread across multiple ones of the memory storage module(s) and/or non-transitory memory storage module(s) storing the one or more databases, depending on the size of the particular database and/or the storage capacity of the memory storage module(s) and/or non-transitory memory storage module(s). In various embodiments, databases can be stored in a cache (e.g., MegaCache) for immediate retrieval on-demand. The one or more databases can each comprise a structured (e.g., indexed) collection of data and can be managed by any suitable database management systems configured to define, create, query, organize, update, and manage database(s). Exemplary database management systems can include MySQL (Structured Query Language) Database, PostgreSQL Database, Microsoft SQL Server Database, Oracle Database, SAP (Systems, Applications, & Products) Database, IBM DB2 Database, and/or NoSQL Database.

Meanwhile, communication between videoconference server 101, adaptive communication server 102, participant devices 103, 104, and/or the one or more databases can be implemented using any suitable manner of wired and/or wireless communication. Accordingly, system 100 can comprise any software and/or hardware components configured to implement the wired and/or wireless communication. Further, the wired and/or wireless communication can be implemented using any one or any combination of wired and/or wireless communication network topologies (e.g., ring, line, tree, bus, mesh, star, daisy chain, hybrid, etc.) and/or protocols (e.g., personal area network (PAN) protocol(s), local area network (LAN) protocol(s), wide area network (WAN) protocol(s), cellular network protocol(s), powerline network protocol(s), etc.). Exemplary PAN protocol(s) can comprise Bluetooth, Zigbee, Wireless Universal Serial Bus (USB), Z-Wave, etc.; exemplary LAN and/or WAN protocol(s) can comprise Institute of Electrical and Electronics Engineers (IEEE) 802.3 (also known as Ethernet), IEEE 802.11 (also known as WiFi), etc.; and exemplary wireless cellular network protocol(s) can comprise Global System for Mobile Communications (GSM), General Packet Radio Service (GPRS), Code Division Multiple Access (CDMA), Evolution-Data Optimized (EV-DO), Enhanced Data Rates for GSM Evolution (EDGE), Universal Mobile Telecommunications System (UMTS), Digital Enhanced Cordless Telecommunications (DECT), Digital Advanced Mobile Phone System (AMPS) (IS-136/Time Division Multiple Access (TDMA)), Integrated Digital Enhanced Network (iDEN), Evolved High-Speed Packet Access (HSPA+), Long-Term Evolution (LTE), WiMAX, etc. The specific communication software and/or hardware implemented can depend on the network topologies and/or protocols implemented, and vice versa. In many embodiments, exemplary communication hardware can comprise wired communication hardware including, for example, one or more data buses, such as, for example, universal serial bus(es), one or more networking cables, such as, for example, coaxial cable(s), optical fiber cable(s), and/or twisted pair cable(s), any other suitable data cable, etc. Further exemplary communication hardware can comprise wireless communication hardware including, for example, one or more radio transceivers, one or more infrared transceivers, etc. Additional exemplary communication hardware can comprise one or more networking components (e.g., modulator-demodulator components, gateway components, etc.).

FIG. 2 is a block diagram of a method 200 for dynamically adapting a communication apparatus based on detected health data according to aspects of this disclosure.

The method 200 begins at block 201. The method 200 may be initiated from the commencement of use of the communication apparatus by a user. For example, the method 200 may be initiated by the beginning of a video call, teleconference, virtual meeting, and the like. In other embodiments, the method 200 may be initiated ad-hoc by a user, such as by the user turning on a setting within their communication apparatus.

Once the method 200 is initiated, at block 203, the system may perform a data check to determine whether or not the user has a profile in the system. For example, the user may have a profile in a user profile database. Each profile in the user profile database can contain specific disabilities and/or interaction preferences for each user of the communication apparatus. Specific disabilities may include ADHD, autism, visual and hearing impairments, anxiety, cerebral palsy, muscular dystrophy, dyslexia, schizophrenia, etc. Interaction preferences may include configurations to customize the digital environment of the communication apparatus to provide a personalized and accessible user experience for the specific user.

If a user profile exists for the user, the method 200 proceeds to block 205, where the system can retrieve the affiliated user's profile. If the profile does not exist for the user, the method 200 proceeds to block 207, where the system can create a profile for the user.

At block 209, the system can customize the environment. The system can customize the digital environment of the communication apparatus based on information in the user profile in order to provide a personalized and accessible user experience. By proactively adjusting the environment, the user can receive timely and context-aware support, such as focus prompts, customized visuals, and adjusted audio cues upon initiation of use of the communication apparatus.

At block 211, the system can perform data collection. For example, the system may collect data via one or more sensors associated with the communication apparatus, such as camera, microphone, webcam, laptop, keyboard, mouse, wearable device, etc.

For data collected via a webcam, at block 213, the system may capture facial expressions of the user and at block 215, may process the webcam data with Open Computer Vision Library (OpenCV). At block 217, after processing the data, the system can evaluate the facial expressions. For example, the facial expressions may indicate the user is experiencing cognitive difficulty based on a furrowed brow, squinting eyes, and the like.

For data collected via a microphone, at block 219, the system may capture voice tones and at block 221, may analyze the voice tones with DeepSpeech. At block 223, after processing the data, the system can evaluate the voice tones. For example, the voice tones of the user may indicate a conversational tone, a formal tone, an edgy tone, a coarse tone, etc. In some embodiments, a more negative tone may indicate that the user is experiencing cognitive difficulty.

For data collected via a laptop, at block 225, the system may capture eye movements and at block 227, may analyze the eye movements with dlib. At block 229, after processing the data, the system can evaluate the eye movements. Eye movements may include blinking, pupil dilation, movement of the position of the eye, etc. Abnormal eye movements, such as short fixation durations or irregular visual scanning patterns, may indicate that the user is experiencing cognitive difficulty.

At block 231, the system can further analyze the data evaluated at block 217, block 223, and block 229 using AI analysis. More specifically, AI may use the data from the previous blocks to identify discomfort in the user.

At block 233, based on the AI analysis of block 231, the system can detect discomfort in the user. Discomfort may include a focus deficit (e.g., difficulty concentrating), memory challenge (e.g., inability to remember certain things), sensory overload (e.g., the user's brain is overwhelmed by information taken in by the five senses), communication difficulty (e.g., difficulty producing clear speech, stuttering, misarticulating words, difficulty understanding spoken language, etc.), auditory challenge (e.g., difficulty with hearing), poor vision (e.g., difficulty with seeing), cognitive overload (e.g., amount of information or tasks is over the amount the user's working memory can handle), limited mobility (e.g., user's ability to move around freely is impaired), or control precision difficulty (e.g., user is unable to effectively adjust the controls of the communication apparatus or other machine).

If discomfort is detected, the method 200 proceeds to block 235, where the system can adjust the digital environment of the communication apparatus. Adjusting the digital environment may include adjusting the display (e.g., screen color, screen contrast, font size) or audio output of the system, and/or adding captioning, navigation aids, or augmented reality prompts to the display. More specifically, the system can perform visual adjustments at block 237 by changing screen settings. The system may also perform audio adjustments at block 239 by modifying audio outputs. If discomfort is not detected, the method 200 proceeds to block 231, where it can continue monitoring the collected data from the sensors.

At block 243, the system can continue to monitor user engagement to determine if further adjustments may be helpful for the user and optionally, repeat the process described above if so. At block 245, the method 200 ends.

FIG. 3 is a block diagram depicting various methods for providing assistance to a user experiencing cognitive strain within UC tools according to aspects of this disclosure. The method 300 may be initiated from the commencement of use of the UC tool by a user. For example, the method 300 may be initiated by the beginning of a video call, teleconference, virtual meeting, and the like. In other embodiments, the method 300 may be initiated ad-hoc by a user, such as by the user turning on a setting within their UC tool.

First, at block 301, the system may perform emotion-sensitive feedback and cognitive load balancing. To do this, at block 303, the system can collect emotional and behavioral data from sensors in the UC tools. For example, the system may collect data via one or more sensors associated with the UC tool, such as a camera, microphone, webcam, laptop, keyboard, mouse, wearable device, etc. The emotional and behavioral data captured from the sensors may include a variety of types of data. For instance, for data collected via a microphone, the data may include voice tones. For data collected via a laptop, the data may include eye movements. For data collected via a webcam, the data may include facial expressions of the user.

At block 305, the system can process data using AI or machine learning (ML) for emotion detection. The system can monitor emotional cues from users during conversations, using AI to detect stress, confusion, or frustration. For example, the system may process the webcam data with Open Computer Vision Library (OpenCV), which can evaluate the facial expressions in the webcam data of the user. The facial expressions may indicate the user is experiencing cognitive difficulty based on a furrowed brow, squinting eyes, and the like. In another example, the system may analyze the voice tones from the microphone data with DeepSpeech. The voice tones of the user may indicate a conversational tone, a formal tone, an edgy tone, a coarse tone, etc. In some embodiments, a more negative tone may indicate that the user is experiencing cognitive difficulty. As another example, the system may analyze the eye movements from the webcam data with dlib. Eye movements may include blinking, pupil dilation, movement of the position of the eye, etc. Abnormal eye movements, such as short fixation durations or irregular visual scanning patterns, may indicate that the user is experiencing cognitive difficulty.

Other possible emotional or mental states may include a focus deficit (e.g., difficulty concentrating), memory challenge (e.g., inability to remember certain things), sensory overload (e.g., the user's brain is overwhelmed by information taken in by the five senses), communication difficulty (e.g., difficulty producing clear speech, stuttering, misarticulating words, difficulty understanding spoken language, etc.), auditory challenge (e.g., difficulty with hearing), poor vision (e.g., difficulty with seeing), cognitive overload (e.g., amount of information or tasks is over the amount the user's working memory can handle), limited mobility (e.g., user's ability to move around freely is impaired), or control precision difficulty (e.g., user is unable to effectively adjust the controls of the UC tool or other machine).

At block 307, the system can adjust cognitive interventions, such as memory interventions and cognitive prompts, based on the emotional state analysis to avoid overwhelming the user. For instance, if rising cognitive stress is detected through voice or facial cues, the system may delay complex memory exercises and provide simpler prompts instead, so that cognitive load is balanced with the user's emotional state.

At block 309, the system can create real-time memory aids and adaptive memory exercises. The system may create real-time memory aids that are contextually relevant and tailored to the user's communication flow. These aids can retrieve information from past conversations, providing personalized memory prompts to help users recall key points or tasks.

At block 311, the system can detect memory gaps during conversations in the UC tool. For example, based on the user's speech, the system may identify that a user is having difficulty remembering a word, phrase, or fact.

At block 313, the system can provide real-time memory aids based on past conversations. For example, the system may evaluate previous conversations of the user from a memory log and determine the word, phrase, or fact the user may be trying to remember. The system may offer subtle prompts to remind them of the information they are forgetting during live sessions to keep users engaged and cognitively active.

Additionally, at block 315, the system can integrate adaptive memory exercises, such as personalized recall prompts and reminders, designed to reinforce memory retention, triggered during or after communication sessions. These exercises help users stay focused on the present conversation and improve memory performance over time.

If the system detects a memory gap, at block 317, the system may further use Explainable AI (XAI) to ensure trust and transparency. XAI is a way to help users understand how the AI algorithm makes decisions. For example, the XAI may explain the reasoning behind which interventions are used and how and when the interventions are used.

At block 319, the system can use XAI techniques to explain AI actions to users. At block 321, the system can provide clear explanations, from XAI, for memory aids, cognitive interventions, or memory exercises. This may help users understand the logic behind AI-driven actions, improving engagement with the system. For example, if a memory aid is suggested during a meeting, the system can explain why it chose that moment to intervene based on the user's communication patterns and emotional cues. The XAI module can use techniques like LIME (Local Interpretable Model-agnostic Explanations) or SHAP (SHapley Additive exPlanations) to generate explanations for the AI's decisions. For example, when a memory prompt is offered, the system generates an explanation like: "This reminder was suggested because you previously had difficulty recalling this point during similar conversations." This may be displayed as a tooltip or verbal explanation, depending on user preferences.

Where the system may provide emotional cues, at block 323, the system may utilize a customizable cognitive companion. The cognitive companion can act as the user's interface, offering a personalized experience based on their cognitive abilities, preferences, and communication style and real-time support.

At block 325, the cognitive companion can offer real-time, context-specific memory and emotional prompts. The cognitive companion can interact with the user throughout communication sessions, delivering real-time memory aids, adaptive memory exercises, emotional feedback, and cognitive load balancing interventions.

At block 327, the user may personalize the companion based on the user's preferences and needs. Users can customize the companion's behavior, tone, and level of intervention to suit their specific cognitive needs, ensuring appropriate, timely, and personalized support. This adaptability can make the system effective for users with varying degrees of cognitive impairment, providing a flexible, user-centric experience.

FIG. 4 illustrates a flow diagram of a method 400 for determining a user's detected cognitive or sensory state from monitored health data in a communication apparatus and adapting the communication apparatus based on the user's detected cognitive or sensory state. The method 400 may be triggered upon initiation of a session of the communication apparatus (e.g., video call, voice conference, virtual meeting etc.) or triggered ad-hoc by a user.

The method 400 begins at block 401, where the system may retrieve, from a user profile database, a user profile associated with the user, the user profile comprising one or more specific disabilities and a plurality of interaction preferences of the user. Specific disabilities may include ADHD, autism, visual and hearing impairments, anxiety, cerebral palsy, muscular dystrophy, dyslexia, schizophrenia, etc. The interaction preferences may include configurations to customize the digital environment of the communication apparatus to provide a personalized and accessible user experience for the specific user. The user profile database may be a database within the system configured to store comprehensive profiles for each user of the communication apparatus detailing specific disabilities (if any) and interaction preferences of the respective users. However, in some embodiments, upon initial use of the system by a user, the database may not yet contain a user profile. As such, in that case, the system can create a new profile for the user and, as the system processes the user's data, update the profile accordingly.

At block 403, the system may receive, via one or more sensors, real-time physiological and behavioral data of the user. The sensors may include a plurality of input devices, such as camera, microphone, webcam, laptop, keyboard, mouse, or wearable device. The sensors may be configured to capture detailed physiological and behavioral data including eye movement, facial expressions, skin color, eye color, and voice tone of the user. The system may use various sub-processes to collect the physiological and behavioral data. For instance, the system may apply OpenCV to process images to capture and analyze facial expressions and eye movements. In embodiments, the system may use DeepSpeech to analyze voice tones. In embodiments, the system may employ the dlib toolkit for eye movements, facial landmark detection, and expression analysis. In embodiments, the system may also use a monitoring module to track user engagement through metrics such as typing speed and mouse movements, which can facilitate continuous assessment of user responsiveness.

At block 405, the system may analyze, using a machine learning model, the physiological and behavioral data to determine a cognitive or sensory state of the user. The system may use various sub-processes to analyze the data. For instance, various different machine learning models may be employed to analyze the various types of data, such as a computer vision model, a voice analysis model, or a face detection model. The system may use an AI analysis engine to process the collected data in real-time to assess the current cognitive and sensory states of the user, identifying any signs of discomfort or distraction. The AI analysis engine is configured to use sophisticated algorithms to detect specific needs aligned with identified disabilities, based on the behavioral data analyzed. For example, the system may deploy DeepSpeech to convert speech to text and analyze voice tones. In embodiments, the system may analyze behavioral data such as examining eye movements, facial expressions, and voice tones to evaluate cognitive and sensory states in real-time. The system may use a computer vision model to analyze facial expressions in images from the physiological and behavioral data. Alternatively, or in addition, the system may use a voice analysis model to track tone, pitch, or speech rates of speech from the physiological and behavioral data. The system may also use a face detection model to analyze eye movements in images from the physiological and behavioral data. The system may further use AI to identify discomfort or distraction in users based on the analyzed data, which may indicate when the cognitive impairment is triggered. The discomfort or distraction may include focus deficit, memory challenge, sensory overload, communication difficulty, auditory challenge, poor vision, cognitive overload, limited mobility, or control precision. However, this is not meant to be limiting or required. In other embodiments, the system can analyze the physiological and behavioral data to determine discomfort or distraction of the user using a computerized algorithm and without the use of AI.

In some embodiments, the system may further determine a cognitive state of the user by comparing the physiological and behavioral data to historical physiological and behavioral data of the user. For example, the system can identify that the user is experiencing discomfort or distraction when the current physiological and behavioral data deviates from the historical physiological and behavioral data. In this way, the system can identify behavior of the user or physiological changes that is atypical compared to historical behavior or physiology. Comparing to historical data may also reduce erroneous identification of discomfort or distraction by comparing to historical behavior or physiology of the user. As an example, on a given day analyzed in isolation, a user's eye color or skin color may not indicate discomfort or distraction. However, when compared to historical data, the system may identify a change in eye or skin color that may indicate a vision impairment or onset of dementia.

At block 407, the system may identify, based on the determined cognitive or sensory state and the user profile, one or more specific needs of the user. The specific user needs maybe include adaptations to the communication apparatus related to the user's cognitive impairment to tailor the system's response to the discomfort or distraction in the particular user based on the data analysis results and the user profile.

At block 409, the system may modify, based on the one or more identified specific needs, one or more digital environment elements of the communication apparatus to address the cognitive or sensory state of the user, such as screen colors, contrast, font size, audio output, captioning, navigation aids, or augmented reality prompts through a response unit configured to enhance visual clarity and reduce visual strain. For example, the system can adapt audio outputs by modulating pitches and volumes and include features such as real-time captioning to assist users with hearing impairments. Additionally, the system can implement tailored interactive elements like augmented reality (AR) prompts and simplified navigation aids to improve user interaction and ease of use. Each adaptive element, whether visual, auditory, or interactive, may be designed to comply with ETSI EN 301 549 or other accessibility standard.

As a comprehensive example, the system may retrieve a user profile for a user in a video meeting, showing that the user has Autism. The system may further receive video data from a webcam showing the user covering their ears and closing their eyes. Based on that, the system may determine that the user is experiencing sensory overload. Therefore, the system can determine that the cognitive or sensory state is a sensory overload. Based on the determination that the user is experiencing sensory overload and the user has Autism, the system may identify that the specific needs of the user may be to simplify visuals and lower saturation colors of the display, modulate pitch and tone of voices, suppress harsh sounds in the audio, minimize user interface (UI) complexity, simplify buttons and icons of the display, control animation pacing, and/or avoid flashing visuals. The system can then adapt the communication apparatus according to those specific needs. A full list of possible adaptations of the communication apparatus based on the impairment and cognitive state is listed below in Table 1.

Table 1 provides a comprehensive breakdown of how the adaptive communication system responds to specific impairments associated with various cognitive, sensory, and physical disorders. Each impairment may be matched with tailored actions (such as visual enhancements, auditory support, UI modifications, and environmental adjustments) to create a more accessible user experience in real-time. Where applicable, the system's actions may conform to established accessibility standards like Web Content Accessibility Guidelines (WCAG), Section 508, EN 301 549, and ISO 9241-171.

In instances where the standards do not fully cover the required accommodations, the system can introduce novel enhancements, addressing previously unaddressed areas and advancing accessibility in digital collaboration contexts.

Table 1 indicates how the adaptive communication system can conform to specific impairments associated with various cognitive, sensory, and physical disorders. Each impairment may be matched with tailored actions (e.g., Keys such as visual enhancements, auditory support, UI modifications, and environmental adjustments) to create a more accessible user experience in real-time. The table is structured to provide granular, impairment-specific solutions while complying with or extending beyond the current standards.

**Table 1**

| **Impairment** | **Visual Enhancements** | **Auditory Support** | **UI Modifications** | **Environmental Adjustments** |
|---|---|---|---|---|
| **ADHD:** Focus/Attention Deficit | Highlight key info | Subtle auditory cues | Dynamic prompts to maintain engagement | Adjust work session pacing |
| | Adaptive colors and contrast adjustments | Modulate volume for attention triggers | Minimize distractions | Calibrate response feedback |
| **ADHD:** Memory Challenges | Use simplified visuals | Auditory reminders for important items | Contextual visual prompts | Timely pauses to allow info consolidation |
| | Progressive disclosure of info | | to recap key points | |
| **Autism:** Sensory Overload | Simplified visuals | Modulate pitch and tone of voices | Minimize UI complexity | Controlled animation pacing |
| | Low-saturation colors | Suppress harsh sounds | Simplified buttons and icons | Avoid flashing visuals |
| **Autism:** Communication Difficulty | Use of symbols/icons instead of text | Simple tones for notifications | Social story integration into UI | Calibrate to communication preferences |
| | | Auditory feedback reduction | Simplified navigation | Enable emoji feedback |
| **Hearing Impairment:** Auditory Challenges | Scalable typography | Real-time captioning | Visual feedback on microphone activity | Audio-to-visual conversion |
| | Clear visual cues for sound events | Amplify speech clarity with filters | Text-based notifications | Simplified speech cues in noisy environments |
| **Visual Impairment:** Poor Vision | High contrast | Auditory descriptions for visual content | Screen reader integration | Progressive disclosure of content to avoid overload |
| | Large fonts | | Text-to-speech support | |
| | Scalable interface | | | |
| **Anxiety:** Sensory Overwhelm | Minimize color complexity | Soft, non-intrusive notification sounds | Less distracting interface | Reduce visual clutter |
| | Control animation speed | | Focus mode with reduced elements | Use of calming colors and elements |
| | Visual simplicity | Gentle auditory prompts | Simplified interactions | Controlled environment for consistency |
| **Schizophrenia:** Cognitive Overload | Text-to-symbol conversion for ease | Reduce sudden noises | Use large, clear navigation cues | Limit dynamic changes |
| **Cerebral Palsy:** Limited Mobility | Large, easily clickable interface elements | Voice commands for interface navigation | Touch-to-voice conversion | Auto-adaptive pacing of interaction responses |
| | | | Simplified input options | |
| **Muscular Dystrophy:** Control Precision | High-contrast large buttons | Voice-guided commands for critical actions | Touchscreen-based controls | Dynamic interface control depending on user movements |

FIG. 5 illustrates a flow diagram of a method 500 for generating cognitive prompts for a user based on the user's mental state. The method 500 may be initiated from the commencement of use of the UC tool by a user. For example, the method 500 may be initiated by the beginning of a video call, teleconference, virtual meeting, and the like. In other embodiments, the method 500 may be initiated ad-hoc by a user, such as by the user turning on a setting within their UC tool.

The method 500 may begin at block 501, where the system may receive, via one or more sensors, real-time physiological and behavioral data of a user. Sensors integrated into Unified Communication (UC) tools may collect real-time physiological and behavioral data. The sensors may include a plurality of input devices, such as camera, microphone, webcam, laptop, keyboard, mouse, or wearable device. The sensors may be configured to capture detailed physiological and behavioral data including eye movement, facial expressions, skin color, eye color, and voice tone of the user. Physiological and behavioral data may further include heart rate and skin conductance data from the wearable devices, voice data including tone, pitch, and speed of speech, and/or images including facial expressions and eye movements. Voice analysis algorithms can track tone, pitch, and speech rate of voice data, while computer vision algorithms analyze facial expressions in the image data. Wearable devices could gather heart rate and skin conductance data to detect stress levels. In embodiments, the system may also use a monitoring module to track user engagement through metrics such as typing speed and mouse movements, which can facilitate continuous assessment of user responsiveness.

At block 503, the system can categorize, using an emotion recognition module, the physiological and behavioral data into one or more mental states. An emotion recognition module may be a type of machine learning module. For example, the emotion recognition module may be a machine learning model such as a convolutional neural network (CNN) for image data analysis (facial expressions) or a recurrent neural network (RNN) or transformers for voice data analysis. The emotion recognition module may process the input and categorize it into emotional or mental states (e.g., stress, frustration, etc.).

At block 505, the system can analyze, using a natural language processor, the content of the physiological and behavioral data to create a memory log from past conversations. Natural language processing (NLP) may analyze the content (e.g., speech) of communication sessions, including during live conversations. The system can create memory logs from past conversations and use this data to track when a user is showing signs of forgetting, such as asking repetitive questions. These logs may be indexed for fast retrieval during live sessions. If a keyword is recognized as something the user has struggled to recall before, the system can fetch the relevant memory aid from prior logs using a search engine optimized for speed (e.g., Elasticsearch).

At block 507, the system can determine or predict, using the memory log and the one or more mental states, the user is experiencing cognitive strain. Cognitive strain may include forgetfulness, attention deficits, stress, processing difficulties, or cognitive fatigue. For example, using the memory log and the one or more mental states, the system may determine when the user is showing signs of forgetting because the user is asking the same questions repeatedly and experiencing frustration. The system can apply predictive models based on task complexity (e.g., the length of conversations, number of simultaneous tasks) and physiological signs to predict cognitive strain. For example, heart rate variability (HRV) could indicate stress, and task-switching behavior could signal cognitive overload.

At block 509, the system can identify, based on the determined cognitive strain, cognitive prompts for the user by accessing a predefined library of memory aids ranked by complexity. For example, the cognitive strain may include stress or confusion. When the AI detects stress or confusion, it dynamically modulates the cognitive prompts in real-time by accessing a predefined library of memory aids ranked by complexity. For example, if the AI detects stress based on facial cues, it may select simpler memory aids such as basic reminders rather than detailed recaps. The system may further identify other interventions for the user based on the determined cognitive strain, as described in FIG. 2.

As another example, the system may identify cognitive prompts by recognizing, using NLP, a word that the user has struggled with before (e.g., by looking in the memory log) and retrieve a relevant memory aid to the word from the prior memory logs using a search engine optimized for speed.

At block 511, the system can send the identified cognitive prompts (or other identified intervention) to the user. These aids can be displayed unobtrusively on the screen, such as a sidebar in the communication interface, or read out loud if the user is participating in a voice or video call. The aids may be ranked based on context relevance and time of need using contextual matching algorithms.

After a conversation, the system may introduce exercises based on memory gaps identified during the session. For instance, if the user failed to recall names or tasks, a memory reinforcement module may automatically generate flashcard-style quizzes or a short activity focused on recalling these details. These exercises can adapt over time, getting harder as the user improves their recall, and may be tracked using performance metrics.

In some embodiments, the system may use a customizable cognitive companion. During communication sessions, the companion can operate in the background, providing interventions when triggered. When the companion detects memory gaps or cognitive strain, it may offer context-specific suggestions that can adapt to the flow of conversation. For instance, if the user forgets a participant's name during a call, the companion can discreetly highlight the name on the screen or whisper the name via an audio cue.

In addition, the system may tailor its response based on the type of cognitive impairment. For a detected memory impairment (e.g., short term memory loss), the system may use real-time memory aids. In this case, the AI can retrieve memory logs and provide prompts in real-time to help recall key points. The system may further use NLP and AI memory retrieval algorithms. The system can determine a memory impairment is occurring by identifying gaps in communication or failure to recall names. The system can improve the user experience or interface interaction by providing visual aids in a sidebar or audio prompts. The system may adapt or personalize the response by making the prompts more complex as memory improves.

For a detected attention deficit, the system may perform cognitive load balancing. In this case, the system can adjust complexity of tasks based on detected stress/fatigue. The system may further use emotion recognition via voice/facial analysis and/or AI stress detection. The system can determine an attention deficit is occurring by detecting high cognitive load or frustration. The system can improve the user experience or interface interaction by simplifying the user interface (UI) and/or hiding non-essential information from the user. The system may reduce the complexity of interventions in response to user stress.

For a detected cognitive failure, the system may perform emotion-sensitive feedback. In this case, the system can monitor emotional state and adapt memory exercises accordingly. The system may further use AI emotion analysis using physiological and behavioral cues. The system can determine a cognitive failure is occurring by detecting fatigue or confusion in the user. The system can improve the user experience or interface interaction by adapting UI responses, such as creating a calming tone and/or reducing task complexity. The system may further learn user patterns and delays or simplify tasks based on fatigue history.

For detected stress-related cognitive decline, the system may perform adaptive memory exercises. In this case, the system can automatically deliver exercises post-session based on memory gaps. The system may further use AI-driven cognitive exercises, and/or adaptive learning. The system can determine stress-related cognitive decline is occurring by identifying memory lapses during communication sessions. The system can improve the user experience or interface interaction by creating flashcard-style quizzes or real-time interactive exercises. The system may further evolve the exercises based on performance data and user feedback.

The system may further adjust the emotion recognition module based on the identified cognitive prompts. For example, the AI may use reinforcement learning to continuously adjust its decision-making. Over time, it can learn which prompts are most effective at different stress levels and memory capabilities of the user and adjust its decision-making accordingly.

FIG. 6 illustrates a block diagram of a system 600 that can be employed for dynamically adapting a communication apparatus based on detected health data, as described in greater detail below. System 600 is merely exemplary and embodiments of the system are not limited to the embodiments presented herein. System 600 can be employed in many different embodiments or examples not specifically depicted or described herein. In some embodiments, certain elements or modules of system 600 can perform various procedures, processes, and/or activities. In these or other embodiments, the procedures, processes, and/or activities can be performed by other suitable elements or modules of system 600.

Generally speaking, system 600 can be implemented with hardware and/or software. Part or all of the hardware and/or software implemented in system 600 can be conventional or part or all of the hardware and/or software can be customized (e.g., optimized) for implementing part or all of the functionality of system 600 described herein. When implemented as software, one or more elements of system 600 can be emulated (e.g., reproduced functionally and/or by action via software). For example, a virtual machine having one or more elements described below can be instantiated on one or more elements of system 100 (FIG. 1).

When implemented as hardware, one or more of the elements of system 600 can be coupled together using one or more chassis configured to hold one or more circuit boards and/or serial bus(es). These boards and buses allow the various elements of system 600 to communicate amongst each other to accomplish their intended purposes. While elements of system 600 are described below individually, each can also be integrated into one or more chassis, circuit boards, and/or buses of system 600. On the other hand, one or more elements of system 600 can also be removable (e.g., via a PCI slot on a motherboard and/or a USB port). One or more elements of system 600 may also be integrated and/or embedded in a different machine or manufacture. Although specific constructions of boards and buses within system 600 are not shown, it should be understood that their construction can be tied to a form factor selected for system 600.

System 600 can take a number of different form factors based on its implementation. For example, system 600 can be implemented as a desktop computer, a laptop computer, a mobile device, and/or a wearable device as described herein. Further, system 600 can comprise a single computer, a single server, a cluster or collection of computers or servers, or a cloud of computers or servers. Typically, a cluster or collection of servers can be used when the demand on 400 exceeds the reasonable capability of a single server or computer, when a distributed structure for system 600 is desired, and/or when parallel computing is desired.

In many embodiments, system 600 can comprise a processor 601, a memory storage 602, an input device 603, a graphics adapter 604, a display device 605, a graphical user interface (GUI) 606, and/or a network adapter 607.

Generally speaking, processor 601 can comprise any type of computational circuit. For example, processor 601 can comprise a microprocessor, a microcontroller, a controller, a complex instruction set computing (CISC) microprocessor, a reduced instruction set computing (RISC) microprocessor, a very long instruction word (VLIW) microprocessor, a graphics processor, a digital signal processor, application specific integrated circuits (ASICs), etc. Processor 601 can be configured to implement (e.g., run) computer instructions (e.g., program instructions) stored on memory devices in system 600. At least a portion of the program instructions, stored on these devices, can be suitable for carrying out at least part of the techniques and methods described herein. Architecture and/or design of processor 601 can be compliant with any of a variety of commercially distributed architecture families. For example, a processor can have a 32-bit (x86) architecture and/or a 64-bit (x86-64, IA64, and AMD64) architecture. Processor 601 can be configured to perform parallel computing in combination with other elements of system 600 and/or additional processors. Generally speaking, parallel computing can be seen as a technique where multiple elements of system 600 are used to perform calculations simultaneously. In this way, complex and repetitive tasks (e.g., training a predictive software application) can be performed faster and with less processing power than without parallel computing.

Generally speaking, memory storage 602 can comprise non-volatile memory (e.g., read only memory (ROM)) and/or volatile memory (e.g., random access memory (RAM)). The non-volatile memory can be removable and/or non-removable non-volatile memory. Meanwhile, RAM can comprise dynamic RAM (DRAM), static RAM (SRAM), or some other type of RAM. Further, ROM can include mask-programmed ROM, programmable ROM (PROM), one-time programmable ROM (OTP), erasable programmable read-only memory (EPROM), electrically erasable programmable ROM (EEPROM) (e.g., electrically alterable ROM (EAROM) and/or flash memory), or some other type of ROM. Memory storage 602 can comprise non-transitory memory and/or transitory memory. All or a portion of memory storage 602 can be referred to as memory storage module(s) and/or memory storage device(s). Memory storage 602 can have a number of form factors when used in system 600. For example, memory storage 602 can comprise a magnetic disk hard drive, a solid state hard drive, a removable USB storage drive, a RAM chip, etc.

Memory storage 602 can be encoded with a wide variety of computer code configured to operate system 600. For example, portions of memory storage 602 can be encoded with a boot code sequence suitable for restoring system 600 to a functional state after a system reset. As another example, portions of memory storage 602 can comprise microcode such as a Basic Input-Output System (BIOS) operable with elements of system 600. Further, portions of the memory storage 602 can comprise an operating system (e.g., a software program that manages the hardware and software resources of a computer and/or a computer network). The BIOS can be configured to initialize and test components of system 600 and load the operating system. Meanwhile, the operating system can perform basic tasks such as, for example, controlling and allocating memory, prioritizing the processing of instructions, controlling input and output devices, facilitating networking, and/or managing files. Exemplary operating systems can comprise software within the Microsoft^{®} Windows^{®}, Mac OS^{®}, Apple^{®} iOS^{®}, Google^{®} Android^{®}, UNIX^{®}, and/or Linux^{®} series of operating systems.

Input device 603 can be configured to allow a user to interact and/or control elements of system 600. A number of devices and be used as input device 603 alone or in combination. For example, input device 603 can comprise a keyboard, a mouse, a touch screen, a microphone, a camera, etc. Input device 603 can be coupled to other elements of system 600 in a number of ways. For example, input device 603 can be coupled via a Universal Serial Bus (USB) port in a wired and/or wireless manner or via a specialized port (e.g., a PS/2 port) depending on the specific device. User inputs through input device 603 can come in a number of forms. For example, when input device 603 comprises a microphone, user input can be received via voice commands and/or a speech to text software application. As another example, when input device 603 comprises a camera, user input can be received via bodily movements that are captured and interpreted by system 600.

Generally speaking, graphics adapter 604 can be configured to receive and/or generate one or more elements for display on display device 605. Exemplary embodiments of graphics adapter 604 can comprise devices within the NVIDIA^{®} GeForce^{®} and/or the AMD^{®} RX^{®} series of video cards. In many embodiments, a chipset present on graphics adapter 604 can be configured to perform similar, simultaneous computations in a manner more efficient than other chipsets. For example, rendering a 3D scene on graphics adapter 604 can involve repeated geometric calculations performed in parallel to generate the 3D scene. As another example, repeated mathematical calculations involved in training a predictive software application can be performed in parallel on graphics adapter 604 more efficiently thank on processor 601. Display device 605 can receive and display signals from graphics adapter 604. A number of devices can be used as display device 605. For example, display device 605 can comprise a computer monitor, a television, a touch screen display, a heads up display (HUD) medium, etc.

In some embodiments, display device 605 can optionally display graphical user interface (GUI) 606. GUI 606 can be a part of and/or displayed by participant devices 103, 104. With regards to form, GUI 606 can comprise text and/or graphics (image) based user interfaces. For example, GUI 606 can comprise a heads up display (HUD). When GUI 606 comprises a HUD, GUI 606 can be projected onto a medium (e.g., glass, plastic, metal, etc.), displayed in midair as a hologram, and/or displayed on display device 605. GUI 606 can be color, black and white, and/or greyscale. GUI 606 can be implemented as an application running on a computer system, such as computer system 600, videoconference server 101 (FIG. 1), adaptive communication server 102 (FIG. 1), and/or participant devices 103, 104 (FIG. 1). GUI 606 can also comprise a website accessed through a network (e.g., network 120 (FIG. 1)). For example, GUI 606 can comprise a cloud storage website. When GUI 606 allows for modification and/or changes to one or more settings in system 600, it can be referred to as an administrative (e.g., back end) GUI. GUI 606 can also be displayed as or on a virtual reality (VR) and/or augmented reality (AR) system or display. GUI 606 can receive a number of interactions from a user via input device 603. For example, an interaction with a GUI can comprise a click, a look, a selection, a grab, a view, a purchase, a bid, a swipe, a pinch, a reverse pinch, etc.

Network adapter 607 can be configured to connect system 600 to a computer network by wired communication (e.g., a wired network adapter) and/or wireless communication (e.g., a wireless network adapter). Network adapter 607 can be integrated into one or more chassis, circuit boards, and/or buses or be removable (e.g., via a PCI slot on a motherboard). For example, network adapter 607 can be implemented via one or more dedicated communication chips configured to receive various protocols of wired and/or wireless communications.

For simplicity and clarity of illustration, the drawing figures illustrate the general manner of construction, and descriptions and details of some features and techniques may be omitted to avoid unnecessarily obscuring the present disclosure. Additionally, elements in the drawing figures are not necessarily drawn to scale. For example, the dimensions of some of the elements in the figures may be exaggerated relative to other elements to help improve understanding of embodiments of the present disclosure. The same reference numerals in different figures denote the same elements.

The terms "first," "second," "third," "fourth," and the like in the description and in the claims, if any, are used for distinguishing between similar elements and not necessarily for describing a particular sequential or chronological order. It is to be understood that the terms so used are interchangeable under appropriate circumstances such that the embodiments described herein are, for example, capable of operation in sequences other than those illustrated or otherwise described herein. Furthermore, the terms "include," and "have," and any variations thereof, are intended to cover a non-exclusive inclusion, such that a process, method, system, article, device, or apparatus that comprises a list of elements is not necessarily limited to those elements but may include other elements not expressly listed or inherent to such process, method, system, article, device, or apparatus.

The terms "left," "right," "front," "back," "top," "bottom," "over," "under," and the like in the description and in the claims, if any, are used for descriptive purposes and not necessarily for describing permanent relative positions. It is to be understood that the terms so used are interchangeable under appropriate circumstances such that the embodiments of the apparatus, methods, and/or articles of manufacture described herein are, for example, capable of operation in other orientations than those illustrated or otherwise described herein.

The terms "couple," "coupled," "couples," "coupling," and the like should be broadly understood and refer to connecting two or more elements mechanically and/or otherwise. Two or more electrical elements may be electrically coupled together, but not be mechanically or otherwise coupled together. Coupling may be for any length of time, e.g., permanent or semi-permanent or only for an instant. "Electrical coupling" and the like should be broadly understood and include electrical coupling of all types. The absence of the word "removably," "removable," and the like near the word "coupled," and the like does not mean that the coupling, etc. in question is or is not removable.

As defined herein, two or more elements are "integral" if they are comprised of the same piece of material. As defined herein, two or more elements are "non-integral" if each is comprised of a different piece of material.

As defined herein, "real-time" can, in some embodiments, be defined with respect to operations carried out as soon as practically possible upon occurrence of a triggering event. A triggering event can include receipt of data necessary to execute a task or to otherwise process information. Because of delays inherent in transmission and/or in computing speeds, the term "real time" encompasses operations that occur in "near" real time or somewhat delayed from a triggering event. In a number of embodiments, "real time" can mean real time less a time delay for processing (e.g., determining) and/or transmitting data. The particular time delay can vary depending on the type and/or amount of the data, the processing speeds of the hardware, the transmission capability of the communication hardware, the transmission distance, etc. However, in many embodiments, the time delay can be less than approximately one second, two seconds, five seconds, or ten seconds.

As defined herein, "approximately" can, in some embodiments, mean within plus or minus ten percent of the stated value. In other embodiments, "approximately" can mean within plus or minus five percent of the stated value. In further embodiments, "approximately" can mean within plus or minus three percent of the stated value. In yet other embodiments, "approximately" can mean within plus or minus one percent of the stated value.

Although systems and methods for context dependent invocation of predictive software application and data storage have been described with reference to specific embodiments, it will be understood by those skilled in the art that various changes may be made without departing from scope of the invention, as defined by the claims. For the purpose of determining the extent of protection conferred by the claims, due account shall be taken of any element which is equivalent to an element specified in the claims.

## Claims

1. A computerized method for determining a user's detected cognitive or sensory state from monitored health data in a communication apparatus and adapting the communication apparatus based on the user's detected cognitive or sensory state, the method comprising:
retrieving, from a user profile database, a user profile associated with the user, the user profile comprising one or more specific disabilities and a plurality of interaction preferences of the user;
receiving, via one or more sensors, real-time physiological and behavioral data of the user;
analyzing, using a machine learning model, the physiological and behavioral data to determine a cognitive or sensory state of the user;
identifying, based on the determined cognitive or sensory state and the user profile, one or more specific needs of the user; and
modifying, based on the one or more identified specific needs, one or more digital environment elements of the communication apparatus to address the cognitive or sensory state of the user.

2. The computerized method of claim 1, wherein the one or more sensors comprise one or more of: camera, microphone, webcam, laptop, keyboard, mouse, or wearable device.

3. The computerized method of claim 1 or claim 2, wherein the machine learning model comprises a computer vision model to analyze facial expressions in images from the physiological and behavioral data.

4. The computerized method of any preceding claim, wherein the machine learning model comprises a voice analysis model to track tone, pitch, or speech rates of speech from the physiological and behavioral data.

5. The computerized method of any preceding claim, wherein the machine learning model comprises a face detection model to analyze eye movements in images from the physiological and behavioral data.

6. The computerized method of any preceding claim, wherein the cognitive or sensory state comprises a distracted state or state of discomfort of the user.

7. The computerized method of claim 6, wherein the distracted state or state of discomfort comprises one or more of focus deficit, memory challenge, sensory overload, communication difficulty, auditory challenge, poor vision, cognitive overload, limited mobility, or control precision.

8. The computerized method of any preceding claim, wherein the one or more digital environment elements comprise one or more of: screen color, screen contrast, font size, audio output, captioning, navigation aid, or augmented reality prompt.

9. The computerized method of any preceding claim, wherein modifying the one or more digital environment elements comprises changing screen settings.

10. The computerized method of any preceding claim, wherein modifying one or more digital environment elements comprises changing audio output by modulating pitches or volumes of audio.

11. The computerized method of any preceding claim, wherein modifying one or more digital environment elements comprises displaying real-time captioning of the audio for the user.

12. The computerized method of any preceding claim, wherein modifying one or more digital environment elements comprises displaying a memory aid for the user.

13. A system comprising:
one or more sensors integrated into a communication apparatus configured to receive real-time physiological and behavioral data of a user;
a user profile database configured to store a plurality of user profiles; and
a processor configured to:
receive, via the one or more sensors, real-time physiological and behavioral data of the user;
analyze, using a machine learning model, the physiological and behavioral data to determine a cognitive or sensory state of the user;
create, based on cognitive or sensory state of the user, a user profile associated with the user in the user profile database, the user profile comprising one or more specific disabilities and a plurality of interaction preferences of the user;
identify, based on the determined cognitive or sensory state and the user profile, one or more specific needs of the user; and
modify, based on the one or more identified specific needs, one or more digital environment elements of the communication apparatus to address the cognitive or sensory state of the user.

14. The system of claim 13, wherein the machine learning model comprises:
a computer vision model to analyze facial expressions in images from the physiological and behavioral data; and/or.
a voice analysis model to track tone, pitch, or speech rates of speech from the physiological and behavioral data; and/or
a face detection model to analyze eye movements in images from the physiological and behavioral data.

15. A computerized method comprising:
receiving, via one or more sensors associated with a communication apparatus, real-time physiological and behavioral data of the user;
analyzing the physiological and behavioral data to determine a cognitive or sensory state of the user;
identifying, based on the determined cognitive or sensory state, one or more specific needs of the user; and
modifying, based on the one or more identified specific needs, one or more digital environment elements of the communication apparatus to address the cognitive or sensory state of the user.
